# EUROPÄISCHE PATENTANMELDUNG

(11) **EP 1 516 915 A1**
(43) Veröffentlichungstag der Anmeldung: **23.03.2005**
(21) Anmeldenummer: 04020726.8
(22) Anmeldetag: 01.09.2004
(51) Int. Cl.: C11D 1/94, C07C 233/36, C07C 291/04, B01F 17/00, A61K 7/50

(54) **Hochkonzentrierte wässrige Lösung amphoterer Tenside**

(30) Priorität: 22.09.2003 DE 10343730
(71) Anmelder: Clariant GmbH, 65929 Frankfurt am Main (DE)
(72) Erfinder: Jonas, Ekaterina, Dr., 65812 Bad Soden (DE); Aigner, Rudolf, 84556 Kastl (DE); Maier, Guillermo, 84533 Haiming (DE); Meyer, Uwe, 84513 Töging (DE); Strobl, Josef, 84329 Wurmansquick (DE)
(74) Vertreter: Mikulecky, Klaus, Dr.

(57) **Zusammenfassung**

Gegenstand der Erfindung sind hochkonzentrierte wässrige Lösungen eines ersten amphoteren Tensids, insbesondere von Betainen oder Aminoxiden, die ein oder mehrere weitere amphotere Cotenside, die sich strukturell von dem ersten amphoteren Tensid unterscheiden, in Gewichtsmengen von 0,01 % bis 10 %, bevorzugt 0,1 % bis 5 %, besonders bevorzugt 0,5 % bis 3 % bezogen auf die hochkonzentrierte wässrige Lösung enthalten.

## Beschreibung

Die Erfindung betrifft hochkonzentrierte wässrige Lösungen von amphoteren Tensiden mit einem Wirkstoffgehalt dieser Tenside von mindestens 30 Gew.-% die in Gegenwart geringer Mengen eines weiteren amphoteren Tensides als pumpbare Flüssigkeiten vorliegen, sowie deren Verwendung.

Es ist bekannt, dass amphotere Tenside, insbesondere Betaine, in Abhängigkeit von den zu ihrer Herstellung verwendeten Fettsäuren bzw. Fettsäuregemischen oberhalb von 30 Gew.-% an Tensid in Wasser lyotrope kristalline Phasen ausbilden. Diese Phasen sind von fester Konsistenz und verhalten sich wie Festkörper. Sie sind nicht mehr pumpbar und lassen sich für den Anwender schwer handhaben.

Eine Senkung der Viskosität von wässrigen Tensidlösungen kann bekanntermaßen durch Zugabe von Lösemittel, beispielsweise n-Alkoholen oder mehrwertigen Alkoholen erreicht werden. In WO 99/24157 sind Lösungen von Betainen beschrieben, die genau definierte Mengen an Betain, Wasser und Ethanol enthalten.

In EP 560 114 werden wässrige, flüssige Lösungen eines Betains mit einem Festkörpergehalt von mindestens 40 Gew.-% beschrieben, gekennzeichnet durch einen Gehalt an 1 bis 3 Gew.-% einer oder mehrerer gesättigter oder ungesättigter Fettsäuren und 0 bis 4 Gew.-% Glycerin, bezogen auf die Lösung. Fettsäure und Glycerin werden vor oder während der Quaternisierung des tertiären Amins mit Chloressigsäure dem Reaktionsgemisch zugegeben.

In EP-A-353-580 ist beschrieben, dass das Phasenverhalten von Betainen durch Zugabe von nichtionischen Tensiden beeinflusst werden kann, wobei allerdings 3 bis 15 Gew.-% an Cotensid notwendig sind.

Durch Zugabe nichtionischer Tenside zu amphoteren Tensiden werden die Tensideigenschaften verändert, was für den Nutzer nachteilig sein kann. Auch Lösemittel und Fettsäureanteile sind häufig in der Formulierung unerwünscht.

Die Aufgabe der Erfindung besteht darin, möglichst hochkonzentrierte wässrige Lösungen von amphoteren Tensiden, insbesondere Betainen oder Aminoxiden, herzustellen, die pumpbar und leicht zu handhaben sind und keine nichtionischen Cotenside oder flüchtige oder öko-toxikologisch problematische, organische Lösungsmittel enthalten. Die Tensidlösungen sollen so hoch konzentriert sein, dass diese aufgrund des verringerten Wassergehaltes selbstkonservierend sind und lange Zeit lagerstabil bleiben ohne dass bakterielle Zersetzung eintritt.

Überraschend wurde gefunden, dass hochkonzentrierte wässrige Lösungen eines amphoteren Tensides in Gegenwart geringer Mengen eines zweiten, strukturell unterschiedlichen amphoteren Tensids flüssig und unbegrenzt phasenstabil sind.

Gegenstand der Erfindung sind hochkonzentrierte wässrige Lösungen eines ersten amphoteren Tensids, insbesondere von Betainen oder Aminoxiden, die ein oder mehrere weitere amphotere Cotenside, die sich strukturell von dem ersten amphoteren Tensid unterscheiden, in Gewichtsmengen von 0,01 % bis 10 %, bevorzugt 0,1 % bis 5 %, besonders bevorzugt 0,5 % bis 3 % bezogen auf die hochkonzentrierte wässrige Lösung, enthalten. Die Konzentration des ersten amphoteren Tensids kann dabei auf Werte von 30 bis 45, vorzugsweise 30 bis 40 Gew.-% eingestellt werden, wobei die Formulierung flüssig bleibt.

Sowohl als erstes amphoteres Tensid als auch als amphoteres Cotensid kommen insbesondere solche der folgenden Formeln in Frage: 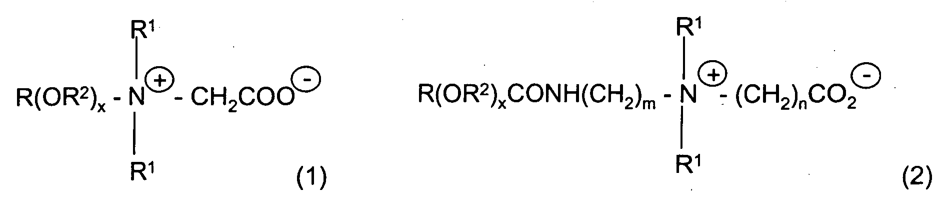 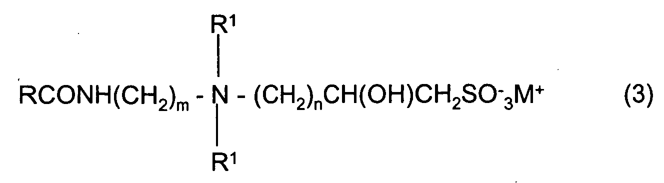 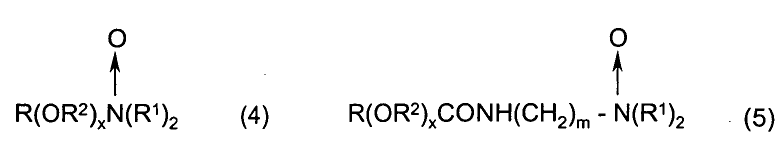 wobei R eine Alkyl-, Hydroxyalkyl- oder Alkylphenylgruppe mit 8 bis 22 Kohlenstoffatomen, jeder Rest R¹ unabhängig voneinander eine Alkyl- oder Hydroxyalkylgruppe mit 1 bis 3 Kohlenstoffatomen bedeutet oder zwei R¹-Gruppen über eine -O- oder -NH-Gruppe unter Ringbildung miteinander verbunden sind, R² eine Alkylengruppe mit 2 bis 3 Kohlenstoffatomen oder Mischungen hiervon, und x eine Zahl von 0 bis 10 bedeuten. M steht für H, Alkalimetall, Erdalkalimetall, Ammonium oder Alkanolammonium und m und n für Zahlen von 1 bis 4. Als Aminoxide sind besonders bevorzugt C₁₀-C₁₈-Alkyldimethylaminoxide, C₈-C₁₂-Alkoxyethyl-Dihydroxyethylaminoxide. Als Betaine bevorzugt sind Verbindungen der Formel (2) mit R¹ = CH₃, m = 3 und n = 1.

Das erfindungsgemäße Verfahren zur Herstellung hochkonzentrierter wässriger Lösungen von Amphotensiden sind auch anwendbar für N-(C₁₂-C₁₈)-Alkyl-β-iminodipropionate, Iminopolyalkanoate als Alkali- und Mono-, Di- und Trialkylammonium-Salze, (C₁₂-C₁₈)-Alkyl-dimethyl-sulfopropyl-betaine, beispielsweise Cocoylamidopropylhydroxysulfobetaine; Amphotenside auf Basis Imidazolin (Handelsname: Miranol® , Steinapon® ), vorzugsweise das Natrium-Salz des 1-(β-Carboxy-methyloxyethyl)-1-(carboxymethyl)-2-lauryl-imidazoliniums, ebenso für Alkyl und Acylglycinate, beispielsweise Cocoylglycinat, Cocoamphoacetate, Cocoamphodiacetate (Cocoamphocarboxyglycinat), Lauroamphoacetat, Cocoamphocarboxypropionate.

Zur erfindungsgemäßen Herstellung hochkonzentrierter wässriger amphoterer Tensidlösungen werden in geringen Mengen amphotere Cotenside zugesetzt.

Als Cotensid eignen sich alle oben genannten amphoteren Tenside mit der Maßgabe, dass sich amphoteres Cotensid und amphoteres erstes Tensid in ihrer Struktur unterscheiden. Als Cotensid können auch Anion-Kation Komplexe, beispielsweise quaternären Ammoniumverbindungen mit Alkylsulfat, Arylsulfat, Alkylsulfonat oder Arylsulfonat eingesetzt werden. Bevorzugte Cotenside sind Verbindungen der Formel (1) mit R¹ = CH₃ und x = 0. Bevorzugt insgesamt sind Mischungen aus Verbindungen der Formel (2) mit R¹ = CH₃, m = 3 und n = 1 oder von Aminoxiden der Formeln 4 oder 5 mit Verbindungen der Formel (1) mit R¹ = CH₃ und x = 0.

Die Herstellung dieser hochkonzentrierten flüssigen Tensidlösungen kann nach zwei Methoden erfolgen.
Zu handelsüblichen wässrigen Tensidlösungen, beispielsweise einer wässrigen Lösung mit 30 Gew.-% Cocoamidopropyl-Betain (® Genagen CAB), werden ein weiteres oder mehrere weitere amphotere/s Cotensid/e bei Raumtemperatur gegeben, das Gemisch 15 Minuten bis 30 Minuten gerührt und anschließend wird der Lösung unter Rühren bei 90 bis 100°C, bevorzugt 95 bis 98°C Wasser entzogen. Die Aufkonzentration der Lösung kann dadurch beschleunigt werden, dass ein Stickstoffstrom an der Oberfläche der Lösung Wasserdampf abführt.
Auf diese Weise können Tensidkonzentrate erhalten werden, die bei Raumtemperatur eine flüssig-viskose Konsistenz bei einem Tensidgehalt WS von 32 bis 38 Gew.-% haben.

Nach einer zweiten Methode können diese hochkonzentrierten Tensidlösungen erhalten werden, indem ein weiteres oder mehrere weitere amphotere/s Cotensid/e bereits dem Reaktionsgemisch bei der Synthese des ersten Tensids zugesetzt wird. Die Synthese der Betaine und Aminoxide erfolgt in bekannter Weise. Eine Modifizierung der Synthesebedingungen ist dabei nicht notwendig.

Die erfindungsgemäßen Tensidlösungen erfüllen die Forderung, frei von nichtionischen Tensiden und organischen Lösungsmitteln zu sein. Sie sind bereits bei dem oben angegebenen Gehalt an amphoterem Cotensid flüssig-viskos.

Die nachfolgenden Beispiele sollen die Erfindung näher erläutern ohne sie darauf einzuschränken:

### Beispiele

### Beispiel 1: Herstellung einer Cocoamidopropylbetainlösung mit einem Wirkstoffgehalt von 38 % in Gegenwart von 1 % Lauryldimethylbetain

212,8 g VE-Wasser, 4,32 g Lauryldimethylbetain-Lösung (30 %ig) (1,0 % m/M bezogen auf Gesamteinwaage) (® Genagen LAB) und 131,6 g Cocos-Amidopropylamin (0,40 Mol) wurden in einem 1 I-Rührkolben vorgelegt und unter Rühren auf 75 bis 80°C erwärmt. Dann wurden 36,5 ml Monochloressigsäure (80 %ig) (103,5 n/n bezogen auf Amidopropylamin) und 21,4 ml Natronlauge (50 %ig) (110 % n/n bezogen auf Amidopropylamin) im Zeitraum von 45 Minuten zugegeben und 30 Minuten bei 75 bis 80°C nachgerührt. Durch Zugabe von 1,1 ml NaOH (50 %ig) wurde der pH-Wert auf 8,0 bis 8,5 eingestellt, die Temperatur innerhalb einer Stunde auf 80 bis 85°C erhöht und 1 Stunde bei 80 bis 85°C gerührt, anschließend innerhalb von 30 Minuten auf 85 bis 90°C erhöht und 1 Stunde nachgerührt und schließlich innerhalb von 30 Minuten auf 90 bis 95°C erhitzt und 5 Stunden bei 90 bis 95°C nachgerührt. Mit 4,0 g Zitronensäure (50 %ig) wurde der pH-Wert auf 5,0 bis 5,5 eingestellt.

### Beispiel 2: Herstellung einer Cocoamidopropylbetainlösung mit einem Wirkstoffgehalt von 37 % in Gegenwart von 0,3 % Lauryldimethylbetain

220,5 g VE-Wasser, 2,2 g Lauryldimethylbetain-Lösung (30 %ig) (0,3 % m/M bezogen auf Gesamteinwaage) (® Genagen LAB) und 131,6 g Cocos-Amidopropylamin (0,40 Mol) wurden in einem 1 I-Rührkolben vorgelegt und unter Rühren auf 75 bis 80°C erwärmt. Dann wurden 36,5 ml Monochloressigsäure (80 %ig) (103,5 n/n bezogen auf Amidopropylamin) und 21,4 ml Natronlauge (50 %ig) (110 % n/n bezogen auf Amidopropylamin) im Zeitraum von 45 Minuten zugegeben und 30 Minuten bei 75 bis 80°C nachgerührt. Durch Zugabe von 1,1 ml NaOH (50 %ig) wurde der pH-Wert auf 8,0 bis 8,5 eingestellt, die Temperatur innerhalb einer Stunde auf 80 bis 85°C erhöht und 1 Stunde bei 80 bis 85°C gerührt, anschließend innerhalb von 30 Minuten auf 85 bis 90°C erhöht und 1 Stunde nachgerührt und schließlich innerhalb von 30 Minuten auf 90 bis 95°C erhitzt und 5 Stunden bei 90 bis 95°C nachgerührt.
Mit 4,0 g Zitronensäure (50 %ig) wurde der pH-Wert auf 5,0 bis 5,5 eingestellt.

### Beispiel 3: Herstellung einer Cocoamidopropylbetainlösung mit einem Wirkstoffgehalt von 38 % in Gegenwart von 1,0% Cocodimethylbetain

206,5 g VE-Wasser, 4,26 g Cocodimethylbetain-Lösung (30 %ig) (1,0 % m/M bezogen auf Gesamteinwaage) (® Genamin CSLB) und 131,6 g Cocos-Amidopropylamin (0,40 Mol) wurden in einem 1 I-Rührkolben vorgelegt und unter Rühren auf 75 bis 80°C erwärmt. Dann wurden 36,5 ml Monochloressigsäure (80 %ig) (103,5 n/n bezogen auf Amidopropylamin) und 21,4 ml Natronlauge (50 %ig) (110 % n/n bezogen auf Amidopropylamin) im Zeitraum von 45 Minuten zugegeben und 30 Minuten bei 75 bis 80°C nachgerührt. Durch Zugabe von 1,1 ml NaOH (50 %ig) wurde der pH-Wert auf 8,0 bis 8,5 eingestellt, die Temperatur innerhalb einer Stunde auf 80 bis 85°C erhöht und 1 Stunde bei 80 bis 85°C gerührt, anschließend innerhalb von 30 Minuten auf 85 bis 90°C erhöht und 1 Stunde nachgerührt und schließlich innerhalb von 30 Minuten auf 90 bis 95°C erhitzt und 5 Stunden bei 90 bis 95°C nachgerührt.
Mit 4,0 g Zitronensäure (50 %ig) wurde der pH-Wert auf 5,0 bis 5,5 eingestellt.

### Beispiel 4: Herstellung einer Cocoamidopropylbetainlösung mit einem Wirkstoffgehalt von 37% in Gegenwart von 0,5% Cocoyldimethylbetain

218,0 g VE-Wasser, 2,19 g Cocodimethylbetain-Lösung (30 %ig) (0,5 % m/M bezogen auf Gesamteinwaage) (® Genamin CSLB) und 131,6 g Cocos-Amidopropylamin (0,40 Mol) wurden in einem 1 I-Rührkolben vorgelegt und unter Rühren auf 75 bis 80°C erwärmt. Dann wurden 36,5 ml Monochloressigsäure (80 %ig) (103,5 n/n bezogen auf Amidopropylamin) und 21,4 ml Natronlauge (50 %ig) (110 % n/n bezogen auf Amidopropylamin) im Zeitraum von 45 Minuten zugegeben und 30 Minuten bei 75 bis 80°C nachgerührt. Durch Zugabe von 1,1 ml NaOH (50 %ig) wurde der pH-Wert auf 8,0 bis 8,5 eingestellt, die Temperatur innerhalb einer Stunde auf 80 bis 85°C erhöht und 1 Stunde bei 80 bis 85°C gerührt, anschließend innerhalb von 30 Minuten auf 85 bis 90°C erhöht und 1 Stunde nachgerührt und schließlich innerhalb von 30 Minuten auf 90 bis 95°C erhitzt und 5 Stunden bei 90 bis 95°C nachgerührt.
Mit 4,0 g Zitronensäure (50 %ig) wurde der pH-Wert auf 5,0 bis 5,5 eingestellt.

### Beispiel 5 Aufkonzentrieren einer Cocoamidopropylbetainlösung durch Abstrippen von Wasser in Gegenwart von Lauryldimethylbetain

500 g Cocoamidopropylbetainlösung (® Genagen CAB 818) wurden unter Rühren bei Umgebungstemperatur mit 4,0 g einer Lösung von Lauryldimethylbetain (® Genagen LAB), entsprechend 1,0 Gew.-% bezogen auf Endeinstellung, versetzt. Der Wirkstoffgehalt der beiden Betainlösungen betrug ca. 30 Gew.-%. Der Kolbeninhalt wurde unter Rühren auf eine Sumpftemperatur von ca. 80°C erwärmt. Bei dieser Temperatur wurde 30 Minuten nachgerührt. Anschließend, wurde der Kolbeninhalt unter Rühren auf ca. 98°C zum Abstrippen von Wasser erwärmt. Um das Abstrippen von Wasser zu beschleunigen, wurde ein leichter Stickstoffstrom über die Oberfläche der Betainlösung geführt. War die berechnete Wassermenge abgestrippt, wurde der Kolbeninhalt unter Rühren auf ca. 60°C abgekühlt und abgefüllt. Von dieser aufkonzentrierten Cocoamidopropyl-betainlösung wurde der Gehalt an Wasser und Kochsalz bestimmt. Auf diese Weise wurden 407 g eines bei Raumtemperatur flüssig-viskosen Cocoamidopropylbetains mit einem Wirkstoffgehalt von 37% erhalten.

### Beispiel 6: Aufkonzentrieren einer Cocosdimethylaminoxidlösung durch Abstrippen von Wasser in Gegenwart von Lauryldimethylbetain (® Genagen LAB)

500 g einer Lösung von Cocosdimethylaminoxid (® Genaminox CST) wurden unter Rühren bei Umgebungstemperatur mit 2,04 g Lauryldimethylbetain-lösung (® Genagen LAB), entsprechend 0,5 Gew.-% bezogen auf Endeinstellung, versetzt. Der Wirkstoffgehalt beider Tensidlösungen betrug ca. 30 Gew.-%. Der Kolbeninhalt wurde unter Rühren auf eine Sumpftemperatur von ca. 80°C erwärmt. Bei dieser Temperatur wurde 30 Minuten nachgerührt. Anschließend wurde der Kolbeninhalt unter Rühren auf ca. 98°C zum Abstrippen von Wasser erwärmt. Um das Abstrippen von Wasser zu beschleunigen, wurde ein leichter Stickstoffstrom über die Oberfläche der Aminoxidlösung geführt. War die berechnete Wassermenge abgestrippt, wurde der Kolbeninhalt unter Rühren auf ca. 60°C abgekühlt und abgefüllt. Von dieser aufkonzentrierten Cocosdimethylaminoxid-lösung wurde der Gehalt an Aminoxid bestimmt. Auf diese Weise wurden 410 g eines bei Raumtemperatur flüssig-viskosen Cocosdimethylaminoxids mit einem Wirkstoffgehalt von 36% erhalten.

Erfindungsgemäß können die oben beschriebenen Tensid-Konzentrate generell in allen Wasch-, Reinigungs-, Desinfektions- und Bleichmitteln jeder Art, insbesondere in Form von wässrigen, wässrig/organischen, insbesondere wässrig/alkoholischen und organischen Formulierungen eingesetzt werden.

In einer weiteren bevorzugten Ausführungsform werden die erfindungsgemäßen Konzentrate in Rinse-off Produkten, bevorzugt Shampoos, Duschbäder, Duschgels und Schaumbäder, eingesetzt.
Die erfindungsgemäßen Mittel können anionische, kationische, nichtionische, zwitter-ionische und/oder weitere amphotere Tenside, sowie Hilfs- und Zusatzstoffe, wie Ölkörper, Emulgatoren und Co-Emulgatoren enthalten. Kosmetische Produkte können gebräuchliche Zusätze, wie z.B. kationische Polymere, Filmbildner, Überfettungsmittel, Stabilisatoren, biogene Wirkstoffe, Glycerin, Konservierungsmittel, Perlglanzmittel, Farb- und Duftstoffe, Lösungsmittel, Lösungsvermittler, Verdickungsmittel, Trübungsmittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen Mitteln antimikrobiell wirkende Agentien zugesetzt werden.
Die erfindungsgemäßen Wasch-, Reinigungs- und Desinfektionsmittel können weitere spezifische Hilfs- und Zusatzstoffe beispielsweise, Salze, Bleichmittel, Bleichaktivatoren, optische Aufheller, Vergrauungsinhibitoren, Konservierungsmittel, Duft- und Farbstoffe, Schauminhibitoren und Sequestriermittel enthalten.

Als anionische Tenside bevorzugt sind (C₁₀-C₂₀)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Bevorzugte kationische Tenside sind quartäre Ammoniumsalze, wie Di-(C₁₀-C₂₄)-Alkyl-dimethylammoniumchlorid oder -bromid, vorzugsweise Di-(C₁₂-C₁₈)-Alkyldimethylammoniumchlorid oder -bromid; (C₁₀-C₂₄)-Alkyl-dimethylethylammoniumchlorid oder -bromid; (C₁₀-C₂₄)-Alkyl-trimethylammoniumchlorid oder -bromid, vorzugsweise Cetyltrimethylammoniumchlorid oder -bromid und (C₂₀-C₂₂)-Alkyl-trimethylammoniumchlorid oder -bromid; (C₁₀-C₂₄)-Alkyl-dimethylbenzylammoniumchlorid oder -bromid, vorzugsweise (C₁₂-C₁₈)-Alkyl-dimethylbenzylammoniumchlorid; N-(C₁₀-C₁₈)-Alkyl-pyridiniumchlorid oder -bromid, vorzugsweise N-(C₁₂-C₁₆)-Alkyl-pyridiniumchlorid oder -bromid; N-(C₁₀-C₁₈)-Alkyl-isochinoliniumchlorid, -bromid oder -monoalkylsulfat; N-(C₁₂-C₁₈)-Alkyl-polyoylaminoformylmethylpyridiniumchlorid; N-(C₁₂-C₁₈)-Alkyl-N-methyl-morpholinium-chlorid, -bromid oder -monoalkylsulfat; N-(C₁₂-C₁₈)-Alkyl-N-ethyl-morpholinium-chlorid, -bromid oder -monoalkylsulfat; (C₁₆-C₁₈)-Alkyl-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N,N-diethyl-N-methylammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Als nichtionische Tenside bevorzugt sind Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics® ); Fettsäurealkylolamide, (Fettsäureamidpolyethylenglykole); N-Alkyl-, N-Alkoxypolyhydroxyfettsäureamid, Saccharoseester; Sorbitester und der Polyglykolether.

Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; N-Acylaminoalkyl-N,N-dimethyl-acetobetain, vorzugsweise N-(C₈-C₁₈)-Acylaminopropyl-N,N-dimethylacetobetain; (C₁₂-C₁₈)-Alkyl-dimethyl-sulfopropyl-betain; Amphotenside auf Basis Imidazolin (Handelsname: Miranol® , Steinapon® ), vorzugsweise das Natrium-Salz des 1-(β-Carboxy-methyloxyethyl)-1-(carboxymethyl)-2-lauryl-imidazoliniums; Aminoxid, z.B. (C₁₂-C₁₈)-Alkyldimethylaminoxid, Fettsäureamidoalkyl-dimethylaminoxid.

Bevorzugte Tenside sind Laurylsulfat, Laurethsulfat, Cocoamidopropylbetain, Natriumcocoylglutamat, Lauroamphoacetat.

Die Mittel können zusätzlich schaumverstärkende Co-Tenside aus der Gruppe der Aminopropionate, Aminoglycinate, Alkanolamide und Polyhydroxyamide enthalten.

Als nichtionogene Co-Emulgatoren kommen u.a. in Betracht Anlagerungsprodukte' von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmonound -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B.

Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, diund tri-Alkylquats und deren polymere Derivate.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVPdimethylaminoethylmethacrylat-Copolymer, Guar-hydroxypropyltriammoniumchloride, sowie Calciumalginat und Ammoniumalginat. Des weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan. Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane, Polyethersiloxan-Copolymere, wie in US 5104 645 und den darin zitierten Schriften beschrieben, die bei Raumtemperatur sowohl flüssig als auch harzförmig vorliegen können.

Geeignete Filmbildner sind, je nach Anwendungszweck wasserlösliche Polyurethane, beispielsweise C10-Polycarbamylpolyglycerylester, Polyvinylalkohol, Polyvinylpyrrolidon, -copolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/ Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carboxyvinyl-Polymere, wie Carbomere und deren Salze, Polysaccharide, beispielsweise Polydextrose und Glucan.

Als Überfettungsmittel können Substanzen wie beispielsweise polyethoxylierte, Lanolinderivate, Lecithinderivate, Polyolfettsäureester, Monoglyceride und Fettsäurealkanolamide verwendet werden, wobei die letzteren gleichzeitig als Schaumstabilisatoren dienen. Als feuchtigkeitsspendende Substanz stehen beispielsweise Isopropylpalmitat, Glycerin und/ oder Sorbitol zu Verfügung.

Als Stabilisatoren können Metallsalze von Fettsäuren, wie z.B. Magnesium-, Aluminium- und/oder Zinkstearat eingesetzt werden.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Als Konservierungsmittel in Betracht kommen beispielsweise Phenoxyethanol, Parabene, Pentandiol oder Sorbinsäure.

Als Farbstoffe können die für kosmetische Zwecke geeigneten und zugelassenen Substanzen verwendet werden.

Als antifungizide Wirkstoffe eignen sich bevorzugt Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Octopyrox.

Um die rheologischen Eigenschaften von wässrigen oder lösungsmittelhaltigen Emulsionen oder Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyvinylamide, Polysulfonsäuren, Polyacrylsäure, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den o.a. Verbindungen, einschließlich ihrer verschiedenen Salze und Ester. Diese Polymere können wahlweise vernetzt oder unvernetzt sein.

Die erfindungsgemäßen Mittel können als Schauminhibitoren Fettsäurealkylesteralkoxylate, Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure, enthalten. Mit Vorteil können auch Gemische verschiedener Schauminhibitoren verwendet werden, z.B. solche aus Silikonöl, Paraffinöl oder Wachsen. Vorzugsweise sind Schauminhibitoren an eine granulare, in Wasser lösliche oder dispergierbare Trägersubstanz gebunden.

Die gewünschte Viskosität der Mittel kann durch Zugabe von Wasser und/oder organischen Lösungsmitteln oder durch Zugabe einer Kombination aus organischen Lösungsmitteln und Verdickungsmitteln eingestellt werden.
Prinzipiell kommen als organische Lösungsmittel alle ein- oder mehrwertigen Alkohole in Betracht. Bevorzugt werden Alkohole mit 1 bis 4 Kohlenstoffatomen wie Methanol, Ethanol, Propanol, Isopropanol, geradkettiges und verzweigtes Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Eine vorteilhafte Mischung aus Lösungsmitteln besteht aus monomerem Alkohol, beispielsweise Ethanol und Polyethylenglykol im Verhältnis 0,5 : 1 bis 1,2 : 1, wobei die erfindungsgemäßen Neutralreinigungsmittel 8 bis 12 Gew.-% einer solchen Mischung enthalten können. Weitere geeignete Lösungsmittel sind beispielsweise Triacetin (Glycerintriacetat) und 1-Methoxy-2-propanol.

Als Verdickungsmittel werden bevorzugt gehärtetes Rizinusöl, Salze von langkettigen Fettsäuren, vorzugsweise in Mengen von 0 bis 5 Gew.-% und insbesondere in Mengen von 0,5 bis 2 Gew.-%, beispielsweise Natrium-. Kalium-, Aluminium-, Magnesium- und Titan-Stearate oder die Natrium und/oder KaliumSalze der Behensäure, sowie Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und diester von Fettsäuren, Polyacrylate, Polyvinylalkohol und Polyvinylpyrrolidon eingesetzt.

Um Spuren von Schwermetallen zu binden, können die Salze von Polyphosphorsäuren, wie 1-Hydroxyethan-1,1-diphosphonsäure (HEDP) und Diethylentriaminpentamethylenphosphonsäure (DTPMP) eingesetzt werden.

Als Perlglanzmittel kommen beispielsweise Glycoldistearinsäureester wie Ethylenglycoldistearat, aber auch Fettsäuremonoglycolester in Betracht.

Als Salze bzw. Stellmittel kommen beispielsweise Natriumchlorid, Natriumsulfat, Natriumcarbonat oder Natriumsilikat (Wasserglas) zum Einsatz.
Als typische Einzelbeispiele für weitere Zusatzstoffe sind Natriumborat, Stärke, Saccharose, Polydextrose, Stilbenverbindungen, Methylcellulose, Toluolsulfonat, Cumolsulfonat, Seifen und Silicone zu nennen.
Nachfolgende Formulierungsbeispiele sollen die Erfindung näher erläutern ohne sie darauf einzuschränken.

Alle Prozentangaben sind Gewichtsprozente. Die Gewichtsprozente beziehen sich auf jeweils 100 % Waschaktivsubstanz.

### Beispiel 1: Neutralreinigungsmittel

### Zusammensetzung:

| | | |
|---|---|---|
| A | ® Genapol LRO flüssig | 12 % |
| | ® Hostapur SAS 60 | 6 % |
| | ® Genapol OA 070 | 5 % |
| | ® Genapol TSM | 3 % |
| B | ® Genagen CAB 818 | 3 % |
| | E-Wasser | ad 100 % |
| | Farbstoff, Konservierungsmittel, Parfüm | q.s. |
| C | Natriumchlorid | 0,7 % |

### Herstellung:

| | |
|---|---|
| I | Die Komponenten von A wurden gemischt |
| II | Zugabe der Komponenten B |
| III | Anschließend wurde mit C die Viskosität eingestellt und gut homogenisiert |

### Beispiel 2: Handgeschirrspülmittel

### Zusammensetzung:

| | | |
|---|---|---|
| A | ® Hostapur SAS 60 | 38,9 % |
| B | E-Wasser | ad 100 % |
| C | ® Genapol LRO paste | 16,7 % |
| | ® Genagen CAB 818 | 16,7 % |
| D | EtOH | 2,8 % |
| | Parfüm, Farbstoff, Konservierungsmittel | q.s. |

### Herstellweise:

| | |
|---|---|
| I | Lösen von A in B |
| II | Nacheinander Zugabe der Komponenten C unter kräftigem Rühren |
| III | Zugabe von D und homogenisieren |

### Beispiel 3: Universalwaschpaste

| | | |
|---|---|---|
| A | ® Genapol LRO liquid | 42,8 % |
| | ® Hostapur SAS 60 | 13,3 % |
| B | E-Wasser | ad 100 % |
| C | ® Hostapon CLG | 8,3 % |
| | ® Genagen CAB 818 | 8,3 % |
| | ® Genapol PGM | 3,0 |
| | Konservierungsmittel, Parfüm | q.s |
| D | NaCl | |

### Herstellweise:

| | |
|---|---|
| I | Mischen der Komponente A |
| II | Zugabe und Lösen von B |
| III | Nacheinander Zugabe der Komponenten C unter kräftigem Rühren |
| IV | Einstellen der Viskosität mit D |

### Beispiel 4: Feinwaschmittel

| | | |
|---|---|---|
| A | ® Hostapur SAS 60 | 9,0 % |
| B | E-Wasser | ad 100 % |
| C | ® Genapol LRO liquid | 35,0 % |
| | ® Genagen CAB 818 | 10,0 % |
| | ® Genapol OA 050 | 1,0 % |
| D | NaCl | 0,8 % |

### Herstellweise:

| | |
|---|---|
| I | Lösen von A in B |
| II | Nacheinander Zugabe der Komponenten C unter kräftigem Rühren |
| III | Einstellen der Viskosität mit D |

### Beispiel 5: Duschbad

| | Komponente | Gew.-% |
|---|---|---|
| 1 | PEG-120 Methyl Glucose Dioleat | 2,25 |
| 2 | Polyquaternium-10 | 0,3 |
| 3 | Glycerin | 2,0 |
| 4 | Polyester | 5,0 |
| 5 | Kokosfettsäure | 2,0 |
| 6 | Medialan LD | 2,0 |
| 7 | Genapol LRO | 3,15 |
| 8 | Genagen LDA | 5,4 |
| 9 | Genagen CAB | 3,0 |
| 10 | Hostapon CLG | 3,6 |
| 11 | Zitronensäure 25 % | 1,05 |
| 12 | Methyldibromoglutaronitril/ Phenoxyethanol | 0,05 |
| 13 | Parfüm | 0,5 |
| 14 | Natrium-Styrol-Acrylat-Copolymer/ Natrium- | |
| | Laurylsulfat/Trideceth-7 | 0,8 |
| 15 | E-Wasser | ad 100 |

Komponente 1, 2, 4 und 5 wurden vorgelegt und in ca. 70°C heißen E-Wasser unter Rühren gelöst. Nacheinander wurden 6, 7, 8, 9, 10 und 3 unter Rühren zugegeben und der pH-Wert mit Zitronensäure auf pH 6,2 eingestellt. Durch Zugabe von 12 und 13 wurde das Mittel konserviert und parfümiert und mit dem Trübungsmittel 14 versehen.

### Beispiel 6: Duschgel

| | Komponente | Gew.-% |
|---|---|---|
| 1 | ®Carbopol ETD 2020 | 1, 5 |
| 2 | ®Polyquaternium-10 | 0,3 |
| 3 | Glycerin | 2,0 |
| 4 | ®Emulsogen SRO | 2,0 |
| 5 | ®Genagen LDA | 9,2 |
| 6 | ®Genagen CAB | 4,0 |
| 7 | ®Hostapon CLG | 4,8 |
| 8 | Zitronensäure | 0,5 |
| 9 | Methyldibromoglutaronitril/ Phenoxyethanol | 0,05 |
| 10 | Parfüm | 0,5 |
| 11 | Trübungsmittel ®Opacifyer 641 | 0,8 |
| 12 | E-Wasser | ad 100 |

Komponente 1 und 2 wurden vorgelegt und in ca. 70°C heißem E-Wasser unter Rühren gelöst. Nacheinander wurden 3, 4, 5, 6 und 7 unter Rühren zugegeben und der pH-Wert mit Zitronensäure auf pH 6,0 eingestellt. Durch Zugabe von 9 und 10 wurde das Mittel konserviert und parfümiert und anschließend mit dem Trübungsmittel 11 versehen.

### Beispiel 7: Duschgel

| | Komponente | Gew.-% |
|---|---|---|
| 1 | ®Carbopol ETD 2020 | 3,0 |
| 2 | ®Polyquaternium-10 | 0,3 |
| 3 | ®Emulsogen SRO | 3,0 |
| 4 | ®Medialan LD | 2,0 |
| 5 | ®Genagen LAA | 7,2 |
| 6 | ®Genagen CAB | 4,0 |
| 7 | ®Hostapon KCG | 6,9 |
| 8 | Milchsäure | 0,5 |
| 9 | Konservierungsmittel | q.s. |
| 10 | Parfüm | q.s. |
| 11 | ®Genapol TSM | 1,0 |
| 12 | E-Wasser | ad 100 |

Komponente 1 und 2 wurden vorgelegt und in ca. 70°C heißem E-Wasser unter Rühren gelöst. Nacheinander wurden 3, 4, 5, 6 und 7 unter Rühren zugegeben und der pH-Wert mit Milchsäure auf pH 6,0 eingestellt. Durch Zugabe von 9 und 10 wurde das Mittel konserviert und parfümiert und anschließend mit dem Seidenglanzmittel 11 versehen.

### Verzeichnis der eingesetzten Produkte

| | |
|---|---|
| ® Carbopol ETD 2020 | (Clariant GmbH) Polyacrylsäure, vernetzt |
| SRO® Emulsogen | (Clariant GmbH) Sorbitester auf Basis von Rapsöl |
| ® Genagen LDA | (Clariant GmbH) Laurylamphodiacetat, Na-Salz |
| ® Genagen LAA | (Clariant GmbH) Laurylamphoacetat, Na-Salz |
| ® Genagen CAB 818 | (Clariant GmbH) Cocoamidopropylbetain |
| ® Hostapon CLG | (Clariant GmbH) Natriumlaurylglutamat |
| ® Hostapon KCG | (Clariant GmbH) Natriumcocoylglutamat |
| ® Medialan LD | (Clariant GmbH) Natriumlauroylsarcosinat |
| ® Genapol TSM | (Clariant GmbH) PEG-3 Distearat, Natriumlaurethsulfat |
| ® Opacifier 641 | Na-Methacrylat-Styrol Copolymer |
| Hostapur® SAS 60: | sekundäres Natriumalkansulfonat (ca. 60 % WAS) |
| Genapol® LRO: | C₁₂-C₁₄-Natriumalkyldiglycolethersulfat (30 %WAS) |
| Genapol® OA 050 | C₁₂-C₁₄-Oxalkoholpolyglykolether mit 5 EO |
| Genapol® OA 070 | C₁₂-C₁₄-Oxalkoholpolyglykolether mit 7 EO |
| Polyester | ca. 40 mol % Terephthalsäure, ca. 10 mol % |
| | Ethylenglykol, ca. 10 mol-% Propylenglycol, ca. 20 mol % |
| | Polyethylenglycol, ca. 10 mol-% Fettalkoholethoxylat, ca. |
| | 10 mol-% Polyol |

## Patentansprüche

1. Hochkonzentrierte wässrige Lösungen eines ersten amphoteren Tensids, enthaltend ein oder mehrere amphotere Cotenside, die sich strukturell von dem ersten amphoteren Tensid unterscheiden, in Gewichtsmengen von 0,01 % bis 10 %, bevorzugt 0,1 % bis 5 %, besonders bevorzugt 0,5 % bis 3%, bezogen auf die hochkonzentrierte wässrige Lösung.

2. Hochkonzentrierte wässrige Lösungen nach Anspruch 1, **dadurch gekennzeichnet, dass** sie Verbindungen der Formeln (1) bis (5) 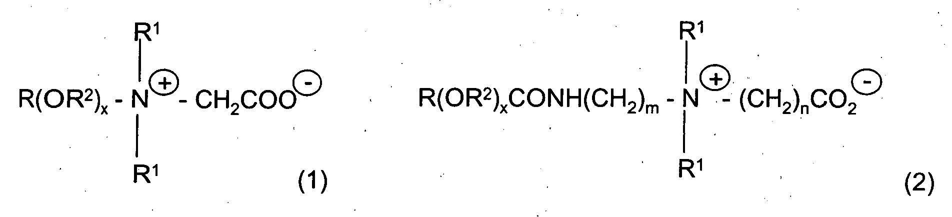 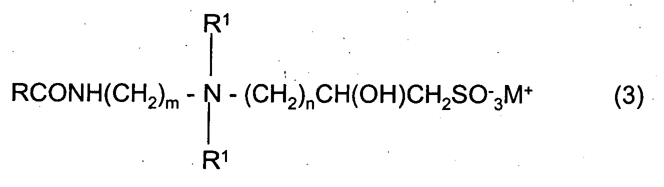 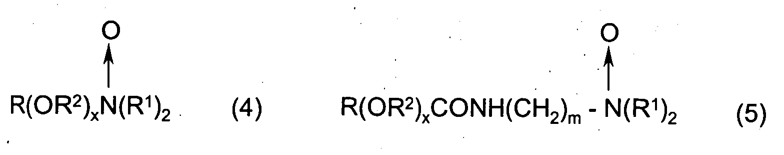 enthalten, wobei
R eine Alkyl-, Hydroxyalkyl- oder Alkylphenylgruppe mit 8 bis 22 Kohlenstoffatomen, jeder Rest R¹ unabhängig voneinander eine Alkyl- oder Hydroxyalkylgruppe mit 1 bis 3 Kohlenstoffatomen bedeutet oder zwei R¹-Gruppen über eine -O- oder -NH-Gruppe unter Ringbildung miteinander verbunden sind, R² eine Alkylengruppe mit 2 bis 3 Kohlenstoffatomen oder Mischungen hiervon, und x eine Zahl von 0 bis 10 bedeuten, M steht für H, Alkalimetall, Erdalkalimetall, Ammonium oder Alkanolammonium und m und n für Zahlen von 1 bis 4, bedeuten.

3. Hochkonzentrierte wässrige Lösungen nach Anspruch 1, **dadurch gekennzeichnet, dass** sie als erstes amphoteres Tensid eine Verbindung der Formel 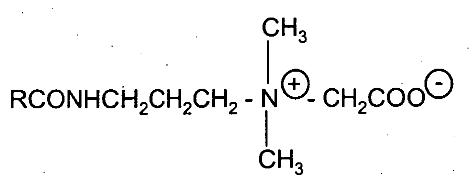 enthalten, wobei R C₈-C₂₂-Alkyl, bedeutet.

4. Hochkonzentrierte wässrige Lösungen nach Anspruch 1, **dadurch gekennzeichnet, dass** sie als Cotensid eine Verbindung der Formel 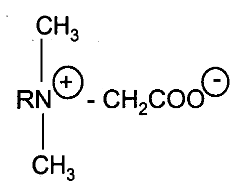 enthalten, wobei R C₈-C₂₂-Alkyl bedeutet.

5. Hochkonzentrierte wässrige Lösungen nach Anspruch 1, **dadurch gekennzeichnet, dass** sie 0,01 bis 10 Gew.-% amphoteres Cotensid, bezogen auf die Lösung sämtlicher Tenside, enthalten.

6. Hochkonzentrierte wässrige Lösungen nach Anspruch 1, **dadurch gekennzeichnet, dass** sie 0,1 bis 5 Gew.-% amphoteres Cotensid, bezogen auf die Lösung sämtlicher Tenside, enthalten.

7. Hochkonzentrierte wässrige Lösungen nach Anspruch 1, **dadurch gekennzeichnet, dass** sie 0,5 bis 3 Gew.-% amphoteres Cotensid, bezogen auf die Lösung sämtlicher Tenside, enthalten.

8. Hochkonzentrierte wässrige Lösungen nach Anspruch 1, **dadurch gekennzeichnet, dass** sie 30 bis 45 Gew.-% des ersten amphoteren Tensids enthalten.

9. Hochkonzentrierte wässrige Lösungen nach Anspruch 1, **dadurch gekennzeichnet, dass** sie 30 bis 40 Gew.-% des ersten amphoteren Tensids enthalten.

10. Kosmetische Mittel, enthaltend eine hochkonzentrierte wässrige Lösung nach Anspruch 1.

11. Wasch-, Reinigungs- und Desinfektionsmittel, enthaltend eine hochkonzentrierte wässrige Lösung nach Anspruch 1.
